# EUROPEAN PATENT APPLICATION

(11) **EP 2 960 840 A1**
(43) Date of publication of application: **30.12.2015**
(21) Application number: 15172005.9
(22) Date of filing: 15.06.2015
(51) Int. Cl.: G06Q 10/06

(54) **COMMUNICATION SYSTEM, MANAGEMENT SYSTEM, INFORMATION MANAGING METHOD, AND CARRIER MEDIUM**

(30) Priority: 26.06.2014 JP 2014131269; 28.04.2015 JP 2015091304
(71) Applicant: Ricoh Company, Ltd., Tokyo 143-8555 (JP)
(72) Inventor: YOSHIDA, Kumiko, Tokyo, 143-8555 (JP)
(74) Representative: Schwabe - Sandmair - Marx

(57) **Abstract**

A management system (50) stores waiting terminal identification information for identifying a waiting communication terminal, for each one of one or more waiting communication terminals that are waiting to start communication with a first communication terminal (10bb). In response to a communication start request for starting communication between the first communication terminal (10bb) and a second communication terminal (10aa), the management system (50) additionally stores identification information for identifying the second communication terminal (10aa) as the waiting terminal identification information of the waiting communication terminal, and transmits information related to waiting that is generated based on the waiting terminal identification information to the second communication terminal (10aa).

## Description

### BACKGROUND

### Technical Field

The present invention generally relates to managing information useful for a communication terminal that is waiting to start communication with a counterpart communication terminal.

### Description of the Related Art

There is an increasing need for communication systems that carryout communication between remotely located sites by transmitting and receiving image data and sound data through a communication network such as the Internet or a dedicated network to perform videoconferencing. For example, such communication system may be used as a remote medical diagnosis system in which a doctor sees and treats a patient at a distance. The patient consults with the doctor through videoconferencing, without going to a hospital. The patient, after consolation, may sometimes need to order prescribed medication to a pharmacy. The patient may then request for prescribed medication through videoconferencing with a pharmacist at the pharmacy.

In case the pharmacist is busy with another patient, the patient needs to wait until his or her turn. Recently, there is a technology to send information regarding how many patients are waiting before the patient to a portable device of the patient. For example, as described in Japanese Patent Application Publication No. 2008-4060, the patient's ID is entered into the pharmacy's system, at the time the patient's order is accepted, at the time of starting preparing the patient's medication, at the time of finishing preparing the patient's medication, etc. While this system provides detailed information to the patient who is waiting, it has been cumbersome to enter the patient's ID for each patient.

### SUMMARY

Example embodiments of the present invention include a management system that stores waiting terminal identification information for identifying a waiting communication terminal, for each one of one or more waiting communication terminals that are waiting to start communication with a first communication terminal. In response to a communication start request for starting communication between the first communication terminal and a second communication terminal, the management system additionally stores identification information for identifying the second communication terminal as the waiting terminal identification information of the waiting communication terminal, and transmits information related to waiting that is generated based on the waiting terminal identification information to the second communication terminal.

Example embodiments of the present invention include a communication system including the above-described management system, and the second communication terminal. The communication system may further include the first communication terminal. The communication system may further include a third communication terminal, which transmits identification information of the first communication terminal to the second communication terminal, before the second communication terminal transmits the communication start request for starting communication with the first communication terminal to the management system.

Example embodiments of the present invention include a method performed by any one of the management system, or any one of the communication terminals.

Example embodiments of the present invention include carrier means such as a recording medium storing a plurality of instructions which, when executed by one or more processors, cause the processors to perform the above-described method.

With the above-described configuration, without requiring a user at the first communication terminal to enter information, a user at the second communication terminal that is waiting to start communication with the first communication terminal is able to obtain information regarding waiting, such as waiting time.

### BRIEF DESCRIPTION OF THE SEVERAL VIEWS OF THE DRAWINGS

A more complete appreciation of the disclosure and many of the attendant advantages and features thereof can be readily obtained and understood from the following detailed description with reference to the accompanying drawings, wherein:
FIG. 1 is a schematic block diagram illustrating a configuration of a communication system according to an example embodiment of the present invention;
FIG. 2 is a perspective view illustrating an outer appearance of a communication terminal of the communication system of FIG. 1, according to an example embodiment of the present invention;
FIG. 3 is a schematic block diagram illustrating a hardware configuration of the communication terminal of FIG. 2;
FIG. 4 is a schematic block diagram illustrating a hardware configuration of any one of a communication management system, a relay device, a program providing system, and a maintenance system of the communication system of FIG. 1, according to an example embodiment of the present invention;
FIG. 5 is a schematic block diagram illustrating a functional configuration of the communication terminal, the management system, and the relay device of the communication system of FIG. 1;
FIG. 6A is an illustration of a terminal management table, managed by the communication management system of FIG. 5;
FIG. 6B is an illustration of a contact list management table, managed by the communication management system of FIG. 5;
FIG. 6C is an illustration of a waiting data management table, managed by the communication management system of FIG. 5;
FIG. 7 is an illustration for explaining transmission or reception of content data and various management information in the communication system of FIG. 1;
FIG. 8 is a data sequence diagram illustrating operation of preparing to start communication, performed by the terminal operated by the doctor in the communication system of FIG. 1, according to an example embodiment of the present invention;
FIG. 9 is an illustration of a counterpart data table, generated by the communication management system of FIG. 5;
FIG. 10 is an example contact list screen displayed at the terminal of the communication system of FIG. 1;
FIG. 11 is a data sequence diagram illustrating operation of requesting to start communication, performed by the terminal operated by the doctor in the communication system of FIG. 1, according to an example embodiment of the present invention;
FIG. 12A is an example screen for display at the terminal based on waiting data;
FIG. 12B is an example screen for display at the terminal based on waiting data;
FIG. 13 is a data sequence diagram illustrating operation of starting communication with the waiting terminal, performed by the terminal operated by the pharmacist in the communication system of FIG. 1, according to an example embodiment of the present invention;
FIG. 14 is an example waiting list screen for display at the terminal;
FIG. 15 is an illustration of a contact list management table, managed by the communication management system of FIG. 5, according to an example embodiment of the present invention;
FIG. 16 is a data sequence diagram illustrating operation of preparing to start communication, performed by the terminal operated by the patient in the communication system of FIG. 1, according to an example embodiment of the present invention; and
FIG. 17 is a data sequence diagram illustrating operation of requesting to start communication, performed by the terminal operated by the patient in the communication system of FIG. 1, according to an example embodiment of the present invention.

The accompanying drawings are intended to depict example embodiments of the present invention and should not be interpreted to limit the scope thereof. The accompanying drawings are not to be considered as drawn to scale unless explicitly noted.

### DETAILED DESCRIPTION

The terminology used herein is for the purpose of describing particular embodiments only and is not intended to be limiting of the present invention. As used herein, the singular forms "a", "an" and "the" are intended to include the plural forms as well, unless the context clearly indicates otherwise. It will be further understood that the terms "includes" and/or "including", when used in this specification, specify the presence of stated features, integers, steps, operations, elements, and/or components, but do not preclude the presence or addition of one or more other features, integers, steps, operations, elements, components, and/or groups thereof.

In describing example embodiments shown in the drawings, specific terminology is employed for the sake of clarity. However, the present disclosure is not intended to be limited to the specific terminology so selected and it is to be understood that each specific element includes all technical equivalents that operate in a similar manner.

In the following description, illustrative embodiments will be described with reference to acts and symbolic representations of operations (e.g., in the form of flowcharts) that may be implemented as program modules or functional processes including routines, programs, objects, components, data structures, etc., that perform particular tasks or implement particular abstract data types and may be implemented using existing hardware at existing network elements or control nodes. Such existing hardware may include one or more Central Processing Units (CPUs), digital signal processors (DSPs), application-specific-integrated-circuits, field programmable gate arrays (FPGAs) computers or the like. These terms in general may be referred to as processors.

Unless specifically stated otherwise, or as is apparent from the discussion, terms such as "processing" or "computing" or "calculating" or "determining" or "displaying" or the like, refer to the action and processes of a computer system, or similar electronic computing device, that manipulates and transforms data represented as physical, electronic quantities within the computer system's registers and memories into other data similarly represented as physical quantities within the computer system memories or registers or other such information storage, transmission or display devices.

### <Configuration of Communication System>

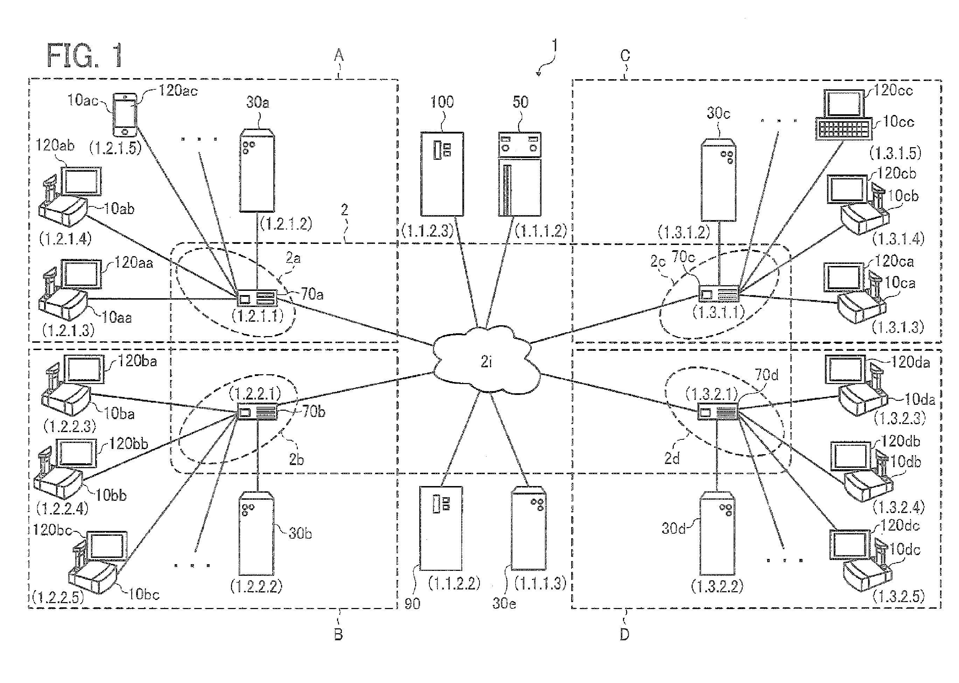

FIG. 1 is a schematic block diagram illustrating a configuration of a communication system 1 according to an example embodiment of the present invention. The communication system 1 of FIG. 1 may be implemented as a data providing system that communicates content data from one communication terminal at one end to a counterpart communication terminal at the other end via a communication management system, or a communication system that allows transmission of data or any information that reflects the feelings of a user, between or among a plurality of communication terminals 10 via a communication management system. Examples of the communication system include, but not limited to, a video communication system such as a videoconference system or a teleconference system, and a personal computer screen sharing system.

In particular, in the following examples, it is assumed that the communication system 1 of FIG. 1 is implemented as the videoconference system. Based on this assumption, the communication management system 50 is implemented as the videoconference communication management system, which is one example of the communication management system. Further, the communication terminal 10 is implemented as the videoconference communication terminal, which is one example of the communication terminal.

The communication system of FIG. 1 includes a plurality of communication terminals 10aa, 10ab, 10ac, 10ba, 10bb, 10bc, 10ca, 10cb, 10cc, 10da, 10db, and 10dc, a plurality of displays 120aa, 120ab, 120ac, 120ba, 120bb, 120bc, 120ca, 120cb, 120cc, 120da, 120db, and 120dc, a plurality of relay devices 30a, 30b, 30c, 30d, and 30e, a communication management system 50, a program providing system 90, and a maintenance system 100.

For the descriptive purposes, in this example, any number of the plurality of communication terminals 10aa to 10dc may be collectively or each referred to as the terminal 10. Any number of the plurality of displays 120aa to 120dc may be collectively or each referred to as the display 120. Any one of the plurality of relay devices 30a to 30e may be collectively or each referred to as the relay device 30. The communication management system 50 may be referred to as the "management system" 50.

The terminal 10 transmits or receives various information to or from the other terminal 10. For example, the terminal 10 establishes a session with the other terminal 10 to transmit or receive content data such as image data or sound data through the established session, thus carrying out videoconference among the plurality of terminals 10.

In the following, image data and sound data are collectively referred to as content data, which is to be transmitted to or from the terminal 10. Alternatively, content data may be text data, or may include sound data, image data, and text data. Further, a moving image, a still image, or both of the still image and the moving image, may be transmitted as the image data.

The management system 50 centrally manages the terminal 10 and the relay device 30. More specifically, the management system 50 establishes a session to transmit content data with the plurality of terminals 10 to perform videoconference among the terminals 10.

The communication system 1 further includes a plurality of routers 70a, 70b, 70c, and 70d, which may be collectively or each referred to as the router 70. The router 70 selects a route that is most suitable for transmitting content data such as image data and sound data. The relay device 30 relays content data among the plurality of terminals 10.

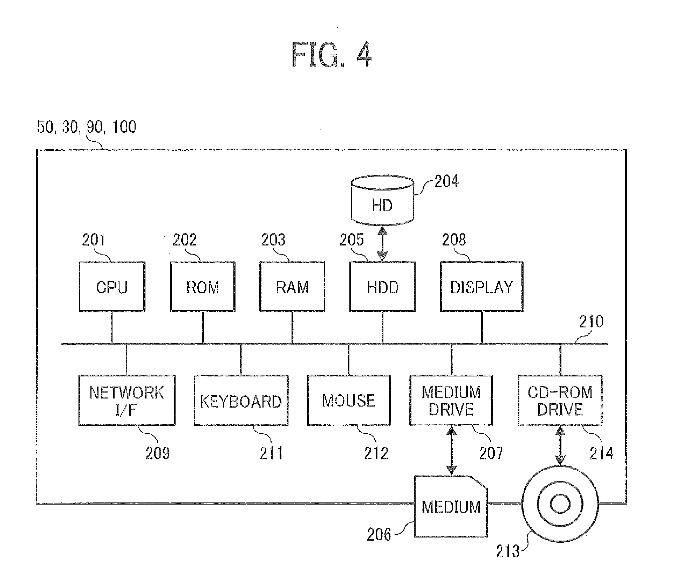

The program providing system 90 includes a hard disk device (HD) 204 (FIG. 4), which stores a terminal control program that causes the terminal 10 to perform various functions or operations. For example, the program providing system 90 sends the terminal control program to the terminal 10 through the Internet 2i to cause the terminal 10 to install the terminal control program. Further, the HD 204 of the program providing system 90 may store a relay control program that causes the relay device 30 to perform various functions or operations. For example, the program providing system 90 sends the relay control program to the relay device 30 through the Internet 2i to cause the relay device 30 to install the relay control program, Further, the HD 204 of the program providing system 90 may store a communication management program that causes the management system 50 to perform various functions or operations. For example, the program providing system 90 sends the communication management program to the management system 50 to cause the management system 50 to install the communication management program.

The maintenance system 100 is a computer system, which may be implemented by one or more computers, to maintain, manage, fix, or upgrade at least one of the terminal 10, relay device 30, management system 50, and program providing system 90. Assuming that the maintenance system 100 is provided within a country, and the terminal 10, relay device 30, management system 50, and program providing system 90 are each installed outside the country, the maintenance system 100 maintains, manages, fixes, or upgrades at least one of the terminal 10, relay device 30, management system 50, and program providing system 90, remotely through the communications network 2. The maintenance system 100 may manage maintenance of at least one of the terminal 10, relay device 30, management system 50, and program providing system 90 without using the communications network 2. For example, a machine type number, a manufacturing number, customer information, maintenance and repair information, and failure log information may be maintained at the maintenance system 100 without using the communications network 2.

Still referring to FIG. 1, the terminals 10aa, 10ab, and 10ac, the relay device 30a, and the router 70a are connected to a local area network (LAN) 2a, at a site "A". The terminals 10ba, 10bb, and 10bc, the relay device 30b, and the router 70b are connected to a LAN 2b, at a site "B".

The terminals 10ca, 10cb, and 10cc, the relay device 30c, and the router 70c are connected to a LAN 2c, at a site "C". The terminals 10da, 10db, and 10dc, the relay device 30d, and the router 70d are connected to a LAN 2d, at a site "D".

In the following, it is assumed that any one of the terminals 10ba, bb, etc. at site B corresponds to the terminal 10 provided at a pharmacy such that the terminal 10 at site B is operated by a pharmacist at the pharmacy. Any one of the terminals 10ca, cc, etc. at site C corresponds to the terminal 10 provided at a hospital such that the terminal 10 at site B is operated by a doctor at hospital. Any one of the terminals 10aa, ab, etc. at site A corresponds to the terminal 10 provided at a location where a patient can easily visit, such that the terminal 10 at site A is operated by the patient. For example, the location at site A may be any public place such as an office, school, or community center, or any private place such as home of the patient. Further, it is assumed that any one of the terminals 10da, db, etc. at site D corresponds to the terminal 10 provided at a location where a patient can easily visit, but which is different than the site A. For this reason, the terminals 10 within the same site may be located at different physical places.

The routers 70a, 70b, 70c, and 70d are connected through the Internet 2i to enable communication between or among the sites that are remotely located from one another.

The management system 50 and the program providing system 90 are connected to the terminal 10 and the relay device 30 through the Internet 2i to be communicable. Any one of the management system 50 and the program providing system 90 may be provided at any one of A, B, C, and D sites, or any other site.

Further, the relay device 30e is connected to the terminal 10 through the Internet 2i.

In this example, the communications network 2 includes the LAN 2a, LAN 2b, Internet 2i, LAN 2c, and LAN 2d. Any one or any portion of these lines or any other lines that may be included in the communications network 2 may be implemented as wired network or wireless network.

As illustrated in FIG. 1, the terminal 10, the relay device 30, the management system 50, the router 70, the program providing system 90, and the maintenance system 100 are each provided with four digit numbers. These four digit numbers separated by dots are the simple expressions of IP addresses respectively assigned to any one of the devices shown in FIG. 1, each of which has a function of communication device. For example, the IP address of the terminal 10aa is "1.2.1.3". For simplicity, it is assumed that the IP address is expressed in IPv4. Alternatively, the IP address may be expressed in IPv6.

### <Hardware configuration of communication system

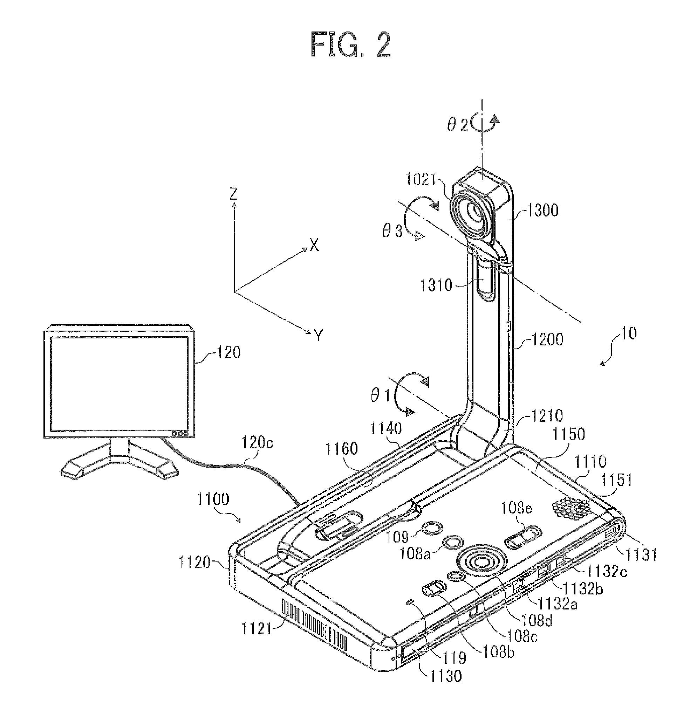

FIG. 2 is a perspective view illustrating the outer appearance of the communication terminal 10 of the communication system 1 of FIG. 1, when the terminal 10 is implemented by a videoconference communication terminal. In FIG. 2, the longitudinal direction of the terminal 10 is referred to as X direction. The direction orthogonal to the X direction, which is the horizontal direction of the terminal 10, is referred to as the Y direction. The direction orthogonal to the X direction and the Y direction is referred to as the Z direction.

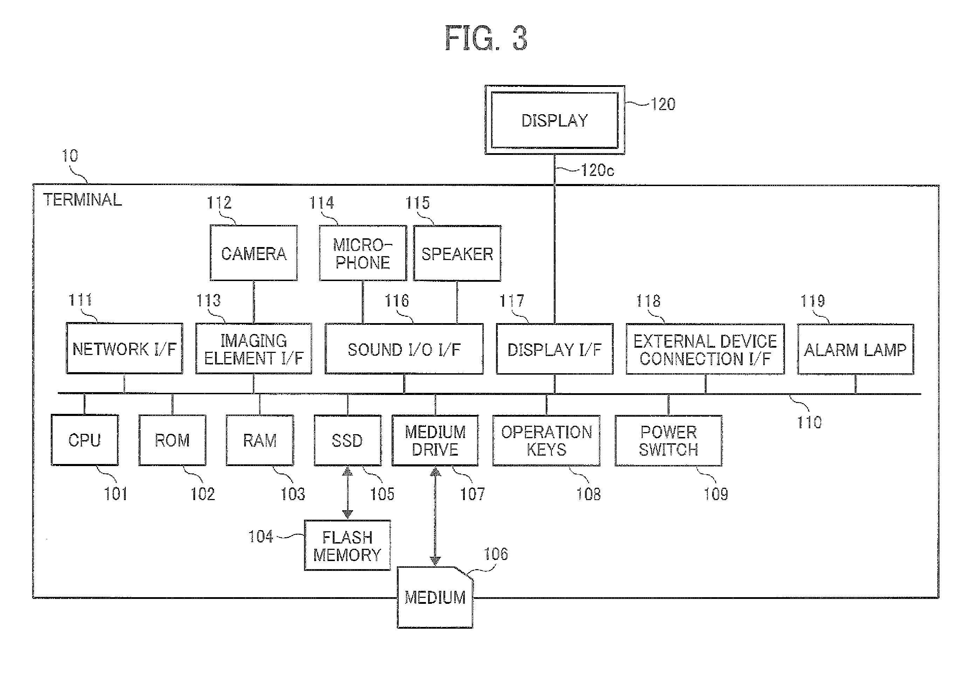

As illustrated in FIG. 2, the terminal 10 includes a body 1100, an arm 1200, and a camera housing 1300. The body 1100 includes a front side wall 1110 having a plurality of air intake holes that are formed over the nearly entire surface of the intake surface of the front side wall 1110. The body 1100 further includes a back side wall 1120 provided with an exhaust surface 1121 having a plurality of exhaust holes over the nearly entire surface of the exhaust surface 1121. When a cooling fan that is provided within the body 1100 is driven, air flows in through the intake holes of the intake surface and out through the exhaust holes of the exhaust surface 1121. The body 1100 further includes a right side wall 1130 formed with a sound pickup hole 1131. Through the sound pickup hole 1131, a microphone 114 (FIG. 3) of the terminal 10 is able to catch sounds such as human voice or any sound including noise.

The body 1100 has an operation panel 1150, which is provided at a front surface toward the right side wall 1130. The operation panel 1150 includes a plurality of operation keys 108a to 108e ("the operation key 108"), a power switch 109, an alarm lamp 119, and a plurality of sound output holes 1151. Through the sound output holes 1151, a speaker 115 (FIG. 3) of the terminal 10 is able to output sounds such as sounds generated based on human voice. The body 1100 further includes a holder 1160, which is provided at the front surface toward the left side wall 1140. The holder 1160, which has a concave shape, accommodates therein the arm 1200 and the camera housing 1300. The right side wall 1130 is further provided with a plurality of connection ports 1132a to 1132c ("connection ports 1132"). The connection ports 1132 allow electrical connection to an external device through an external device connection I/F 118 (FIG. 3). The body 1100 further includes a left side wall 1140, which is provided with a connection port to connect the external display 120 to the display I/F 117 through a cable 120c.

The arm 1200 is attached to the body 1100 via a torque hinge 1210. With the torque hinge 1210, the arm 1200 can be rotated in directions of up and down with respect to the body, while making a tilt angle θ1 of up to 135 degrees. FIG. 2 illustrates the case where the tilt angle θ1 is 90 degrees.

The camera housing 1300 incorporates therein the camera 112 (FIG. 3) that takes an image of an object. The object may be a part of a user, document, or a room where the terminal 10 is located. The camera housing 1300 is provided with a torque hinge 1310. The camera housing 1300 is attached to the arm 1200 through the torque hinge 1310. With the torque hinge 1310, the camera housing 1300 can be rotated with respect to the arm 1200, in the direction of up, down, right, and left, such that the camera housing 1300 is kept at a desired position. More specifically, the camera housing 1300 can be rotated, while making a pan angle θ2 from about -180 degrees to 180 degrees in the direction right and left, and a tilt angle θ3 that ranges from about -45 degrees to +45 degrees in the direction of up and down. In FIG. 2, the pan angle θ2 and the tilt angle θ3 are each 0 degree.

In alternative to the outer appearance of FIG. 2, the communication terminal 10 may be implemented to have any other outer appearance. For example, the communication terminal 10 may look like the general-purpose PC (See "10cc" in FIG. 1), smart phone (See "10ac" in FIG. 1), or tablet. Further, the camera or the microphone does not have to be incorporated into the terminal 10, such that the camera or the microphone that is independent of the terminal 10 may be connected to the terminal 10. Further, when the terminal 10 is implemented by the portable phone, the terminal 10 is connected to the Internet 2i through a wireless network such as wireless LAN or portable phone network. In case the general-purpose information processing apparatus such as the PC or the portable phone is used as the videoconference terminal, a terminal control program is installed with the general-purpose information processing apparatus.

The relay device 30, the management system 50, the program providing system 90, and the maintenance system 100 are each implemented by any desired number of general-purpose computers such as a personal computer or a server computer. For simplicity, explanation of the outer appearance of the computer is omitted.

FIG. 3 is a schematic block diagram illustrating a hardware configuration of the communication terminal 10. As illustrated in FIG. 3, the terminal 10 includes a central processing unit (CPU) 101, a read only memory (ROM) 102, a random access memory (RAM) 103, a flash memory 104, a solid state drive (SSD) 105, a medium drive 107, the operation key 108, the power switch 109, a network interface (I/F) 111, the camera 112, an imaging element interface (I/F) 113, the microphone 114, the speaker 115, a sound input/output interface (I/O I/F) 116, the display interface (I/F) 117, the external device connection interface (I/F) 118, and the alarm lamp 119, which are electrically connected through a bus 110 such as an address bus or data bus. The CPU 101 controls entire operation of the terminal 10. The ROM 102 stores therein a control program for execution by the CPU 101, such as an initial program loader (IPL). The RAM 103 functions as a work area for the CPU 101. The flash memory 104 stores therein various data such as the terminal control program, image data, or sound data such as voice data. The SSD 105 controls reading or writing of various data with respect to the flash memory 104 under control of the CPU 101. The medium drive 107 controls reading or writing of various data with respect to a removable recording medium 106 such as a flash memory. The operation key 108 allows the user to input a user instruction, for example, by allowing the user to select a communication destination such as the counterpart terminal 10. The power switch 109 allows the user to switch on or off the power of the terminal 10. The network I/F 111 allows the terminal 10 to transmit data through the communications network 2.

The camera 112 takes an image of an object to obtain image data under control of the CPU 101. The imaging element I/F 113 controls operation of the camera 112. The microphone 114 catches sounds such as voice of the user at the terminal 10. The speaker 115 outputs sounds such as sounds generated based on voice of the user at the counterpart terminal 10. The sound I/O I/F 116 controls input or output of sound signals such as voice signals with respect to the microphone 114 and the speaker 115 under control of the CPU 101. The display I/F 117 transmits image data to the display 120 under control of the CPU 101. The external device connection I/F 118 controls connection of the terminal 10 to various types of outside device. The alarm lamp 119 is lighted to report a trouble in the function of the terminal 10.

The display 120 may be implemented by a liquid crystal display (LCD) or an organic light emitting displays, which displays various data such as an image of an object or an operation icon. As illustrated in FIGs. 2 and 3, the display 120 is connected to the display I/F 117 through the cable 120c. The cable 120c may be implemented by an analog RCB (VGA) signal cable, a component video cable, a high-definition multimedia interface (HDMI) signal cable, or a digital video interactive (DVI) signal cable.

The camera 112 includes a plurality of devices such as a lens system, and a solid-state image sensing device that photo-electrically converts a light to generate an image of an object. For example, the solid-state image sensing device includes a complementary metal oxide semiconductor (CMOS) or a charge coupled device (CCD).

The external device connection I/F 118 may be connected to an external device such as an external camera, external microphone, or external speaker through a universal serial bus (USB) cable that is connected through the port 1132 of the body 1100 (FIG. 2). When the external camera is connected to the terminal 10, the CPU 101 causes the terminal 10 to capture an image using the external camera, rather than the camera 112 that is incorporated in the terminal 10. When the external microphone or the external speaker is connected to the terminal 10, the CPU 101 causes the terminal 10 to use the external microphone or the external speaker in replace of the incorporated microphone 114 or the incorporated speaker 115.

The recording medium 106, which can be freely attached to or detached from the terminal 10, includes any desired type of recording medium. In alternative to the flash memory 104, any nonvolatile memory that is readable and writable under control of the CPU 101 may be used such as Electrically Erasable and Programmable ROM (EEPROM).

The terminal control program may be written onto a recording medium that is readable by a general-purpose computer such as the recording medium 106 in any format that is installable or executable by a general-purpose computer. Once the terminal control program is written onto the recording medium, the recording medium may be distributed. Further, the terminal control program may be stored in any desired memory other than the flash memory 104, such as the ROM 102.

FIG. 4 illustrates a hardware configuration of the management system 50 of FIG. 1. The management system 50 includes a CPU 201, a ROM 202, a RAM 203, the HD 204, a hard disk drive (HDD) 205, a medium drive 207, a display 208, a network interface (I/F) 209, a keyboard 211, a mouse 212, and a CD-ROM drive 214, which are electrically connected through a bus 210 such as an address bus or a data bus.

The CPU 201 controls entire operation of the management system 50. The ROM 202 stores a control program for execution by the CPU 201, such as an IPL. The RAM 203 functions as a work area for the CPU 201. The HD 204 stores therein various data such as the communication management program. The HDD 205 controls reading or writing of various data with respect to the HD 204 under control of the CPU 201. The medium drive 207 controls reading or writing of various data with respect to a removable recording medium 206 such as a flash memory. The display 208 displays various data such as a cursor, menu, window, character, or image. The network I/F 209 allows the management system 50 to transmit data through the communications network 2. The keyboard 211 includes a plurality of keys, each of which is used for inputting a user instruction through a character, a numeral, or a symbol The mouse 212 allows the user to input a user instruction including, for example, selection or execution of a specific instruction, selection of an area to be processed, and instruction of cursor movement. The CD-ROM drive 214 controls reading or writing of various data with respect to a CD-ROM 213. In alternative to the CD-ROM 213, any removable recording medium may be used.

The communication management program may be written onto a recording medium that is readable by a general-purpose computer such as the recording medium 206 or the CD-ROM 213 in any format that is installable or executable by a general-purpose computer. Once the communication management program is written onto the recording medium, the recording medium may be distributed. Further, the communication management program may be stored in any desired memory other than the HD 204, such as the ROM 202.

The relay device 30 is substantially similar in hardware configuration to the management system 50 of FIG. 4, except for replacement of the management program with a relay device control program that is used for controlling the relay device 30. The relay device control program may be written onto a recording medium that is readable by a general-purpose computer such as the recording medium 206 or the CD-ROM 213 in any format that is installable or executable by the general-purpose computer. Once the relay device control program is written onto the recording medium, the recording medium may be distributed. Further, the relay device control program may be stored in any desired memory other than the HD 204, such as the ROM 202.

The program providing system 90 is substantially similar in hardware configuration to the management system 50 of FIG. 4, except for replacement of the management program with a program providing program that is used for controlling the program providing system 90. The program providing program may be written onto a recording medium that is readable by a general-purpose computer such as the recording medium 206 or the CD-ROM 213 in any format that is installable or executable by the general-purpose computer. Once the program providing program is written onto the recording medium, the recording medium may be distributed. Further, the program providing program may be stored in any desired memory other than the HD 204, such as the ROM 202.

The maintenance system 100 is substantially similar in hardware configuration to the management system 50 of FIG. 4, except for replacement of the management program with a maintenance program that is used for controlling the maintenance system 100. The maintenance program may be written onto a recording medium that is readable by a general-purpose computer such as the recording medium 206 or the CD-ROM 213 in any format that is installable or executable by the general-purpose computer. Once the maintenance program is written onto the recording medium, the recording medium may be distributed. Further, the maintenance program may be stored in any desired memory other than the HD 204, such as the ROM 202.

Other examples of removable recording medium, which may be used in replace of the CD-ROM 213, include, but not limited to, compact disc recordable (CD-R), digital versatile disk (DVD), and blue ray disc.

### <Functional configuration of communication system>

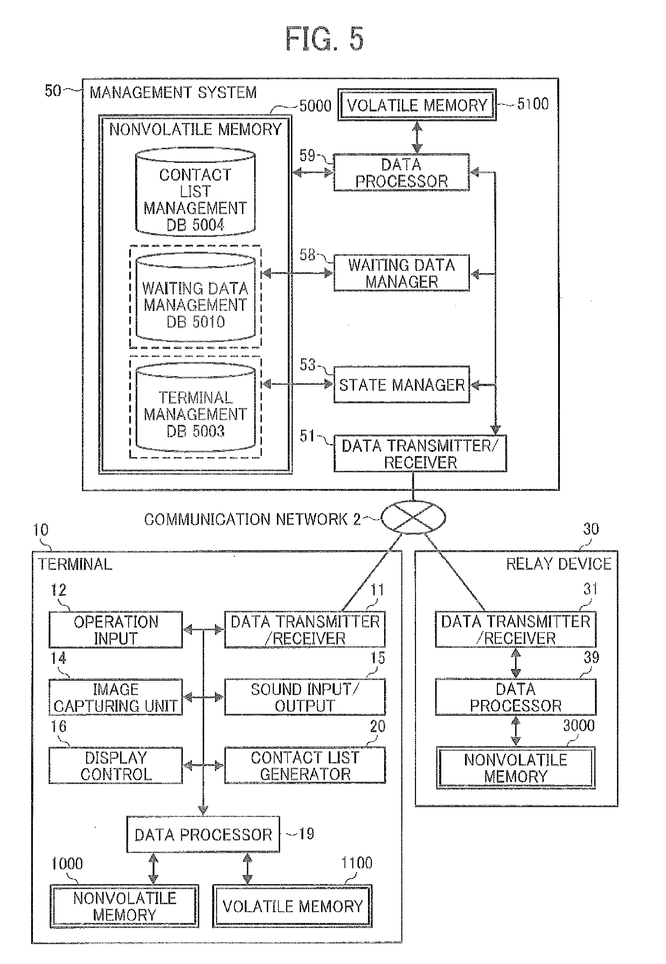

FIG. 5 is a schematic block diagram illustrating functional configuration of the terminal 10, the relay device 20, and the management system 50 of the communication system 1, according to an example embodiment of the present invention. In FIG. 5, the terminal 10, the relay device 30, and the management system 50 communicate data via the communication network 2. For simplicity, in FIG. 5, the program providing system 90 and the maintenance system 100 are omitted.

### <Functional configuration of terminal>

The terminal 10 includes a data transmitter/receiver 11, an operation input 12, an image capturing unit 14, a sound input/output 15, a display control 16, a data processor 10, and a contact list generator 20. In this example, operations or functions that are performed by the functional elements shown in FIG. 5, are performed in cooperation with one or more hardware devices of the terminal 10 that are shown in FIG. 3. More specifically, these elements shown in FIG. 5 correspond to a plurality of functions or functional modules, which are executed according to an instruction of the CPU 101 (FIG. 3) that is generated according to the program being loaded from the flash memory 104 onto the RAM 103. The terminal 10 further includes a volatile memory 1000 implemented by the RAM 103, and a nonvolatile memory 1000 implemented by the flash memory 104 of FIG. 3.

The data transmitter/receiver 11 of the terminal 10, which may be implemented by the network I/F 111, transmits or receives various data or information to or from the other terminal, device, or system through the communication network 2.

The operation input 12, which may be implemented by the operation key 108 or the power switch 109, receives various inputs from a user. For example, when the user turns on the power switch 109, the operation input 12 receives turning on of the power of the terminal 10.

The image capturing unit 14, which may be implemented by the instructions from the CPU 101, the camera 112, and the imaging element I/F 113, captures an image of an object and outputs the captured image as image data.

The sound input/output 15 may be implemented by the sound input/output I/F 116 under control of the CPU 101. In one example, after the microphone 114 converts sounds of the user at the terminal 10 to a sound signal, the sound input/output 15 inputs the sound signal in the form of sound data for further processing. In another example, the sound input/output 15 outputs a sound signal of sound data that is received from the counterpart terminal 10 through the speaker 115.

The display control 16, which may be implemented by the display I/F 117, controls transmission of image data through the external display 120.

The memory control 19 is implemented by the instruction from the CPU 101 or the SSD 105 of FIG. 3. The memory control 19 stores various data in the nonvolatile memory 1000 or volatile memory 1100, or reads out various data from the nonvolatile memory 1000 or volatile memory 1100. The volatile memory 1000 stores therein content data that is received as the terminal 10 communicates with a counterpart terminal 10 such that content data is overwritten. Before the image data is overwritten, an image generated based on the image data is displayed onto the display 120. Before the sound data is output, sounds generated based on the sound data is output through the speaker 115.

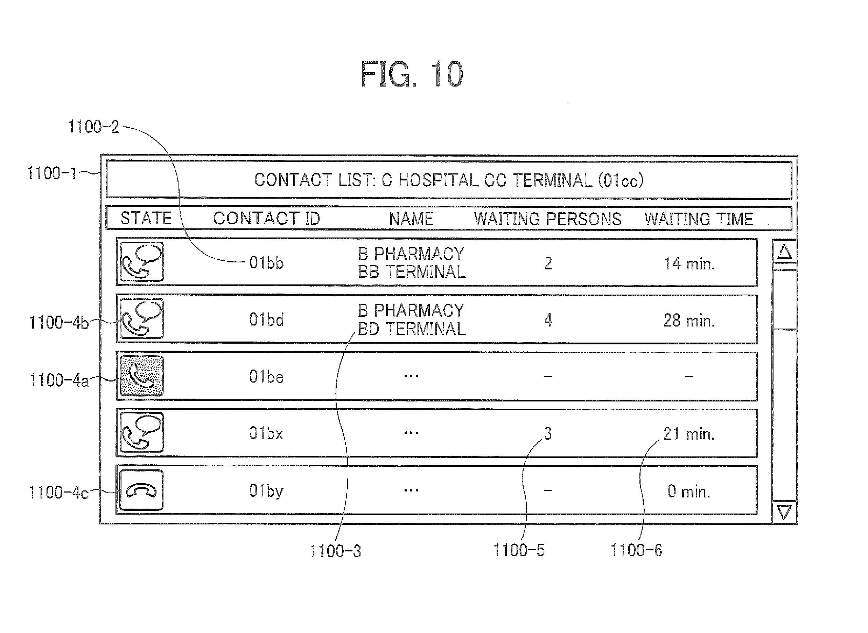

The contact list generator 20 generates or updates a contact list, such as a contact list illustrated in FIG. 10 that shows an icon reflecting a current operation state of a candidate terminal, based on contact list information and a counterpart data table.

### (Functional configuration of relay device)

The relay device 30 includes a data transmitter/receiver 31 and a data processor 39. In this example, operation or functions that are performed by the functional elements shown in FIG. 5, are performed in cooperation with hardware of the relay device shown in FIG. 4. These elements shown in FIG. 5 correspond to a plurality of functions or functional modules, which are executed according to an instruction of the CPU 201 (FIG. 4) that is generated according to the program being loaded from the ROM 202 onto the RAM 203. The relay device 30 further includes a nonvolatile memory 3000 implemented by the HDD 204, which keeps various data or information even after the power of the relay device 30 is turned off.

The data transmitter/receiver 31, which may be implemented by the network I/F 209, transmits or receives various data or information to or from the other terminal, device, or system through the communication network 2.

The data processor 39, which may be implemented by the instructions from the CPU 201 or the HDD 205 of FIG. 3, stores various data in the nonvolatile memory 3000 or reads various data from the nonvolatile memory 3000.

### <Functional configuration of management system>

Still referring to FIG. 5, functional configuration of the management system 50 is explained. The management system 50 includes a data transmitter/receiver 51, a state manager 53, a waiting data manager 58, and a data processor 59. These units shown in FIG. 5 correspond to a plurality of functions or functional modules, which are executed according to an instruction of the CPU 201 (FIG. 4) that is generated according to the communication management program being loaded from the HD 204 onto the RAM 203. The management system 50 further includes a nonvolatile memory 5000 implemented by the HD 204 (FIG. 4) and a volatile memory 5100 implemented by the RAM 203. The nonvolatile memory 5000 keeps various data or information even after power of the management system 50 is turned off.

In this example, a contact ID may be any information used for identifying a communication destination, such as terminal identification for identifying the counterpart terminal 10 or user identification for identifying a user at the counterpart terminal 10. In this example, it is assumed that the contact ID is the terminal ID of the counterpart terminal 10. The relay device ID is any information for identifying the relay device 30 that relays communication with the communication destination such as the counterpart terminal 10. Any one of the contact ID and the relay device ID includes any type of identification information that can be expressed by any language, character, symbol, or mark, or any combination of language, character, symbol, and mark.

### (Terminal management table)

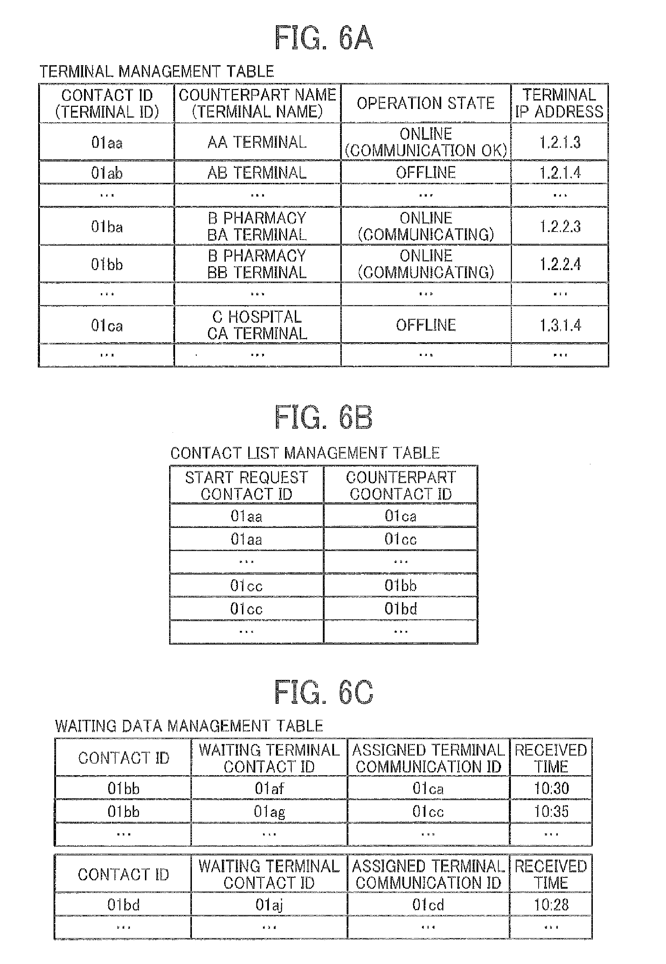

The nonvolatile memory 5000 includes a terminal management DB 5003, which may be implemented by a terminal management table of FIG. 6A. The terminal management table of FIG. 6A stores, for each one of communication terminals 10 managed by the communication system 1, the terminal ID of the terminal 10 that functions as the contact ID, the terminal name of the terminal 10 that functions as a counterpart name to be referred to when the terminal is the counterpart terminal, the operation state of the terminal 10, and the IP address of the terminal 10, in association with one another. The operation state includes, for example, an online state in which the power of the terminal 10 is turned on ("online"), and an offline state in which the power of the terminal is turned off such that the terminal 10 is not available for communication ("offline"). The online state further includes a state in which the terminal 10 is available for communication (online (communication OK)), and a state in which the terminal 10 is communicating (online (communicating)).

### (Contact list management table)

The nonvolatile memory 5000 further includes a contact list management DB 5004, which may be implemented by a contact list management table of FIG. 6B. The contact list management table of FIG. 6B stores, for each one of terminals 10 that can request to start communication with one or more counterpart terminals 10 ("start request terminal"), the contact ID of the start request terminal 10 and a counterpart contact ID of each one of the counterpart terminals 10 in association with each other. For example, the contact list management table of FIG. 6B illustrates that, the start request terminal 10aa having the contact ID "01aa" can request to start communication with any one of the counterpart terminal 10ca having the contact ID "01ca", and the counterpart terminal 10cc having the contact ID "01cc", etc.

### (Waiting data management table)

The nonvolatile memory 5000 further includes a waiting data management DB 5010, which may be implemented by a waiting data management table of FIG. 6C. The waiting data management table of FIG. 6C stores, for each one of the terminals 10 that are managed by the management system 50, a contact ID of the terminal 10 (the terminal 10 of the pharmacist), a contact ID of a waiting terminal 10 (the terminal 10 of the patient) that is waiting to start communication with the terminal 10 as a communication destination, a contact ID of a referrer terminal 10 (the terminal 10 of the doctor) that refers the terminal 10 to the waiting terminal 10, and the received time at which the management system 50 receives a request for starting communication with the terminal 10, in association with one another.

### (Functional configuration of management system)

Referring to FIG. 4, functional configuration of the management system 50 is explained.

The data transmitter/receiver 51, which may be implemented by the network I/F 209, transmits or receives various data or information to the other terminal, device, or system, through the commmication network 2.

The state manager 53, which may be implemented by the instructions from the CPU 201, manages the operation state of the terminal 10 using the terminal management table of FIG. 6A.

The waiting data manager 58, which may be implemented by the instructions from the CPU 201, writes or reads various data to or from the waiting data management DB 5010.

The data processor 59, which may be implemented by the instructions from the CPU 201 that cooperates with a memory such as the HDD 205, writes or reads various data to or from the nonvolatile memory 5000 or the volatile memory 5100.

### <Operation of Communication System>

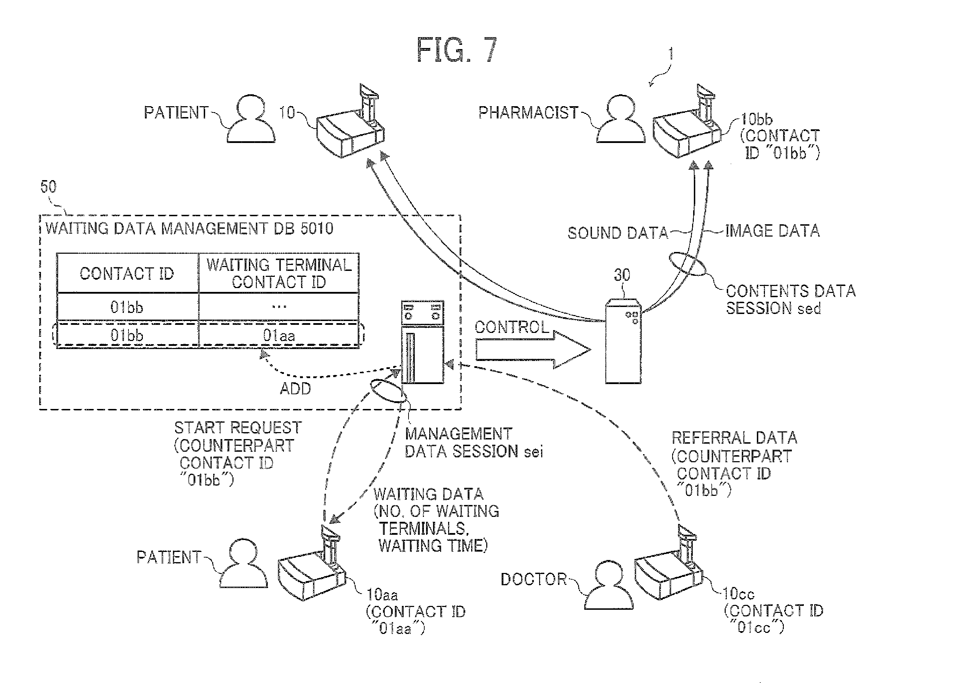

Referring to FIG. 7, operation performed by the comnunication system 1 is explained according to an example embodiment of the present invention. FIG. 7 is an illustration for explaining transmission or reception of data such as content data and various management information in the communication system 1.

As illustrated in FIG. 7, in the communication system 1, a management information session "sei" is established between the terminal 10 and the management system 50 to transmit or receive various management information. Under control of the management system 50, a content data session "send" is established between the terminals 10 via the relay device 30 to transmit or receive content data (image data and/or sound data). More specifically, the content data session "send" transmits or receives content data to carry out videoconference.

The waiting data management DB 5010 of the management system 50 stores a contact ID for each one of one or more terminals 10 (waiting terminals 10, such as the terminals 10 of the patients) waiting to start communication with the terminal 10bb (example of a first communication terminal, such as the terminal 10 of the pharmacist), in association with a contact ID "01bb" of the terminal 10bb. The contact ID can be any identification information used for identifying a communication destination. In this example, the terminal ID "01bb" of the terminal 10bb is used as the contact ID of the terminal 10bb, however, a user ID of a user operating the terminal 10bb may be used instead. The data transmitter/receiver 51 of the management system 50 receives a communication start request for starting communication between the terminal 10bb (the first communication terminal) and the terminal 10aa (example of a second communication terminal, such as the terminal 10 of the patient). When the terminal 10bb is communicating with the other terminal 10, the waiting data manager 58 of the management system 50 adds the contact ID of the terminal 10aa to the waiting data management DB 5010, as the contact ID of the waiting terminal waiting to start communication with the terminal 10bb. The data transmitter/receiver 51 transmits any information that may be useful for the user at the waiting terminal 10aa ("waiting data"), which is obtainable using the identification information of the waiting terminal that is stored in the waiting data management DB 5010. With this configuration, the management system 50 automatically transmits the waiting data that may be usable for the user at the waiting terminal 10aa, as long as the management system 50 adds the contact ID of the waiting terminal 10aa to the waiting data management DB 5010.

In this example, the waiting data is any information related to, or reflects, the act of waiting performed by the waiting terminal 10, which may be useful to the user at the waiting terminal 10. The waiting data management DB 5010 may store various information to be used as waiting data for transmission to the waiting terminal 10aa, such as a number of contact IDs of the waiting terminals 10 waiting to communicate with the same terminal 10bb, or a time period that is calculated based on the number of contact IDs of the waiting terminals 10 for the same terminal 10bb. With this information, the management system 50 obtains the number of waiting terminals 10 for the terminal 10bb that the terminal 10aa is waiting to communicate, the number of persons waiting for communication, and/or the estimated waiting time, to the terminal 10aa.

The contact ID may be identification information for identifying the counterpart terminal 10 as a communication destination, or identification information for a user at the counterpart terminal 10.

For example, in response to receiving selection of the terminal 10bb (Pharmacy: BB terminal) as a communication destination of the terminal 10aa (Patient: AA terminal), at the terminal 10cc (Hospital: CC Terminal), the data transmitter/receiver 51 of the management system 50 receives a communication start request to start communication between the terminal 10aa and the terminal 10bb.

The doctor at the terminal 10cc (Hospital: CC Terminal) thus makes referral for the patient at the terminal 10aa to the pharmacist at the terminal 10bb. That is, the terminal 10cc functions as a "referred terminal", which refers the terminal 10aa to the terminal 10bb, by requesting to start communication between the terminal 10aa and the terminal 10bb.

The data transmitter/receiver 11 at the terminal 10aa receives, from the management system 50, waiting data including such as a number of waiting persons or an estimated waiting time period. The display control 16 at the terminal 10aa outputs the waiting data for display onto the display 120aa. With information based on the waiting data, the user at the terminal 10aa is able to know information regarding, for example, the estimated waiting time period.

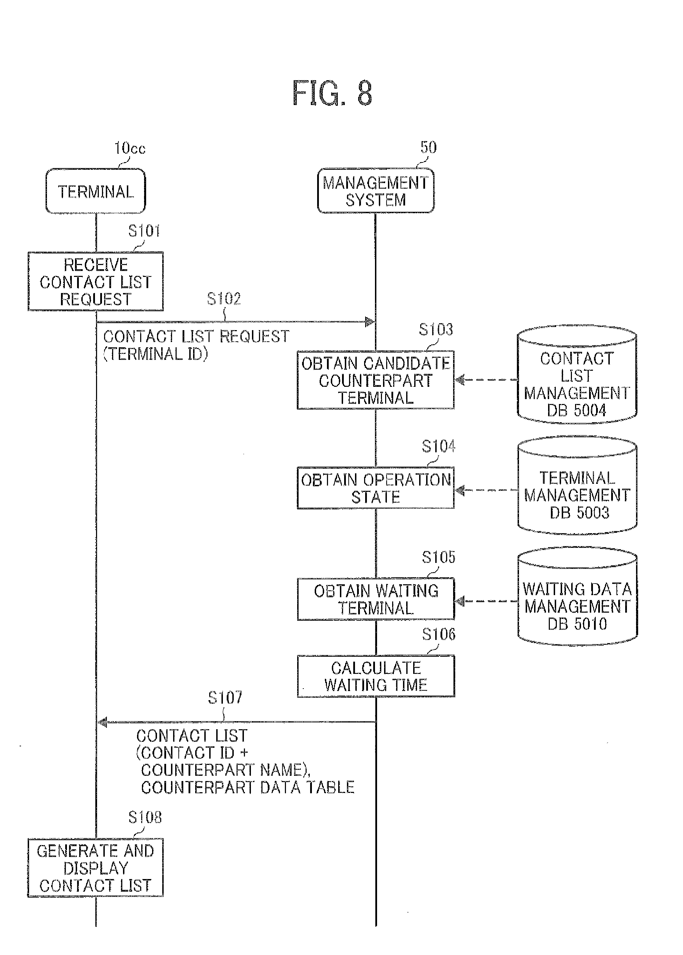

Referring to FIG. 8, operation of preparing to start communication performed by the communication system 1 is explained according to an example embodiment of the present invention. In this example, it is assumed that a content data session "sed" is established between the terminal 10aa and the terminal 10cc via the relay device 30e to transmit or receive content data (image data or sound data).

More specifically, it is assumed that a doctor, who is a user at the terminal 10cc, examines a patient, who is a user at the terminal 10aa, through videoconferencing. After writing a prescription about medication, the doctor presses the operation key 108 of the terminal 10cc to request for a list of candidate counterpart terminals 10 of pharmacists who can prepare medication according to the prescription. In this example, the contact list lists candidates of a terminal 10 at pharmacy that will be a counterpart terminal 10 to start communication with the terminal 10 of the patient. As the operation key 108 is selected at the terminal 10cc, at S101, the operation input 12 of the terminal 10cc receives a contact list request.

At S102, the data transmitter/receiver 11 of the terminal 10cc sends contact list request information ("contact list request"), which requests for contact list, to the management system 50 through the communication network 2. The contact list request information includes the terminal ID "01cc" of the terminal 10cc ("request terminal 10cc").

When the data transmitter/receiver 51 of the management system 50 receives the contact list request information, at S103, the data processor 59 searches the contact list management table of FIG. 6B using the contact ID "01cc" of the request terminal 10cc that sends the contact list request as a search key, to obtain the contact IDs ("01bb", "01bd", etc.) of candidate counterpart terminals 10 that may be selected by the request terminal 10cc as a counterpart terminal for the terminal 10aa.

At S104, the data processor 59 searches the terminal management table of FIG. 6A using the contact ID ("01bb", "01bd", etc.) of each one of the candidate counterpart terminals 10 read at S103, as a search key, to obtain the operation state and the terminal name of the candidate counterpart terminal.

At S105, the data processor 59 of the management system 50 extracts, out of the candidate counterpart terminals 10 read at S103, the contact ID ("01bb", "01bd") of the candidate counterpart terminal 10 having the operation state "online (communicating)". Using the extracted contact ID ("01bb", "01bd") of the candidate counterpart terminal 10 having the operation state "online (communicating)", the data processor 59 reads the contact ID of each one of the waiting terminals 10 that are stored in association with the contact ID of the candidate counterpart terminal 10 from the waiting data management table of FIG. 6C. More specifically, in this example, it is assumed that the contact IDs "01bb" and "01bd" of the candidate counterpart terminals 10bb and 10bd having the operation state "online (communicating)" are extracted.

At S106, the waiting data manager 58 obtains, for the contact ID of each one of the candidate counterpart terminals 10bb and 10bd, a number of contact IDs of the waiting terminals that are obtained at S105. The waiting data manager 58 further calculates, for each one of the candidate counterpart terminals 10bb and 10bd, an estimated waiting time period for the terminal 10 (terminal 10aa) that may request to start communication, based on the number of waiting terminals. For example, a preset time period (for example, 7 minutes) may be multiplied by the number of waiting terminals (or the number of contact IDs of the waiting terminals).

At S107, the data transmitter/receiver 51 transmits contact list information including the contact ID and the terminal name for each one of the candidate counterpart terminals 10, and counterpart data table, to the request terminal 10cc that requests for contact list, through the communication network 2. The counterpart data table includes various information regarding the candidate counterpart terminals 10, such as information obtained at S103 and S104, the number of waiting terminals 10 obtained at S105, and the calculated waiting time period obtained at S106.

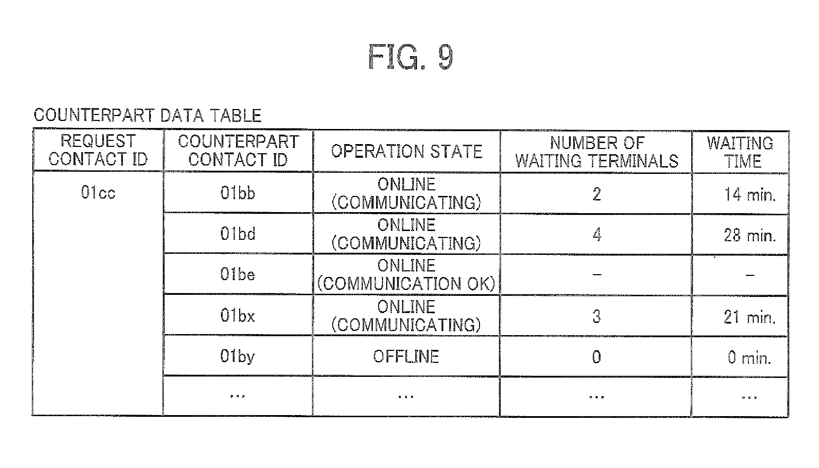

FIG. 9 illustrates an example counterpart data table. The counterpart data table of FIG. 9 stores, a contact ID of the request terminal 10 that requests for contact list, a contact ID of a candidate counterpart terminal 10 for the request terminal 10, the operation state of the candidate counterpart terminal 10, and the number of waiting terminals 10 that are waiting to start communication with the candidate counterpart terminal 10, and the waiting time period, in association with one another.

At S108, the contact list generator 20 of the request terminal 10cc generates a contact list based on the contact list information and the counterpart data table transmitted from the management system 50. The contact list lists the operation state of the candidate counterpart terminal 10, the number of waiting terminals 10 waiting to start communication with the candidate counterpart terminal 10, and the waiting time period, in association with one another. The display control 16 of the request terminal 10cc causes the display 120cc to display the contact list.

FIG. 10 is an example contact list screen. As illustrated in FIG. 10, within a contact list frame 1100-1, the contact list includes, for each one of candidate counterpart terminals 10, a contact ID 1100-2 of the candidate counterpart terminal 10, the counterpart name 1100-3 of the candidate counterpart terminals 10, the icon 1100 (one of icons 1100-4a to 4c) that reflects the operation state of the candidate counterpart terminal 10, the number of waiting persons 1100-5 that correspond to the number of waiting terminals, and the waiting time 1100-6 for the candidate counterpart terminal 10.

Referring to FIG. 10, the contact list generator 20 designates the icon 1100-4a to the candidate counterpart terminal 10 having the operation state "online (communication OK)". The contact list generator 20 designates the icon 1100-4b to the candidate counterpart terminal 10 having the operation state "online (communicating)". The contact list generator 20 designates the icon 1100-4c to the candidate counterpart terminal 10 having the operation state "offline". The terminal 10cc selects at least one of the candidate counterpart terminals 10 having the online state (that is, either the operation state "online (communication OK)" or the operation state "online (communicating)", from the candidate counterpart terminals 10 displayed in the candidate list frame 1100-1, as a communication destination for the terminal 10aa.

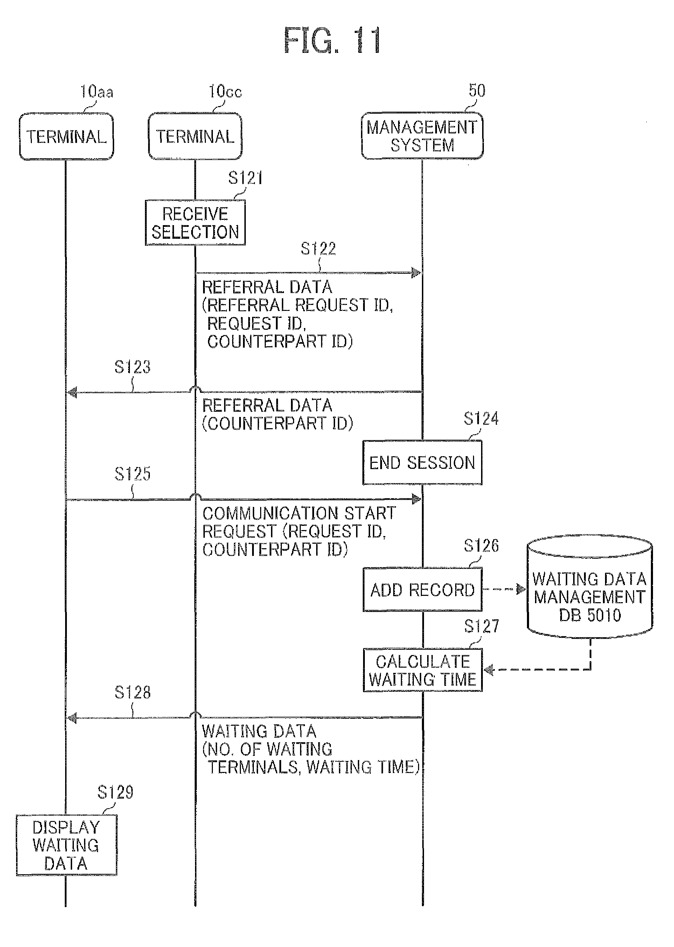

Referring to FIG. 11, operation of requesting the terminal 10aa to start communication with the counterpart terminal 10, which is selected from the contact list displayed through operation of FIG. 8, is explained according to an example embodiment of the present invention.

More specifically, in this example, the doctor at the terminal 10cc, selects the terminal 10bb of the pharmacist, as a communication destination for the patient at the terminal 10aa, to start communication between the terminal 10aa and the terminal 10bb. Thus, the terminal 10cc functions as a referral terminal, which refers the terminal 10aa to the terminal 10bb.

The doctor, who is a user at the terminal 10cc, selects the candidate counterpart terminal 10 having the operation state "online (communication OK)" or the operation state "online (communicating)", from the contact list of FIG. 10 that is displayed at S108, using the operation key 108. The doctor at the terminal 10cc may select the candidate counterpart terminal 10 of the pharmacy in various ways, for example, depending on medication available for that pharmacy, the waiting time (such as the pharmacy having the least waiting time), the location of the pharmacy (such as the pharmacy located closest to the patient site), etc. In response to the selection on the operation key 108, at S121, the operation input 12 of the terminal 10cc receives selection of a counterpart terminal for the terminal 10aa. In the following, it is assumed that the terminal 10bb is selected as the counterpart terminal 10 for the terminal 10aa.

At S122, the data transmitter/receiver 11 of the terminal 10cc transmits referral data to the management system 50, which requests to end a content data session between the terminal 10cc and the terminal 10aa, and to start a content data session between the terminal 10aa and the terminal 10bb. Through sending the referral data to the management system 50, the terminal 10cc operates as a referral terminal 10 that refers the terminal 10aa to the terminal 10bb. More specifically, the referral data includes the contact ID "01cc" of the referral terminal 10cc, the contact ID "01aa" of the terminal 10aa that will be requesting to start communication, and the contact ID "01bb" of the counterpart terminal 10bb to be requested to start communication.

At S123, the data transmitter/receiver 51 of the management system 50 obtains the contact ID "01bb" of the counterpart terminal 10bb, from the referral data that is received, and transmits the obtained contact ID "01bb" of the counterpart terminal 10bb to the terminal 10aa that will start communication with the counterpart terminal 10bb.

At S124, the management system 50 ends the content data session "sed" between the terminal 10cc operated by the doctor and the terminal 10aa operated by the patient. In ending the session, the state manager 52 updates the terminal management table of FIG. 6A, by changing the operation state "online (communicating)" to "online (communication OK)" for each one of the terminals 10aa and 10cc. The operation states of the terminals 10aa and 10cc are specified using the contact IDs "01aa" and "01cc", respectively. Further, in ending the session, the data transmitter/receiver 51 sends a request for ending relaying of content data to the relay device 30 that relays content data between the terminal 10aa and the terminal 10cc. Alternatively, processing to end the content data session may be performed in various other ways using any known method.

After the terminal 10aa leaves from the content data session with the terminal 10cc, at S125, the data transmitter/receiver 11 of the terminal 10aa transmits communication start request information to the management system 50, based on the referral data (that is, the contact ID of the counterpart terminal) received at S123. The communication start request information, which requests to start communication with the counterpart terminal 10bb, includes the contact ID "01aa" of the request terminal 10aa and the contact ID "01bb" of the counterpart terminal 10bb.

In the following, it is assumed that the counterpart terminal 10bb is communicating with the other terminal 10, when the data transmitter/receiver 51 of the management system 50 receives the communication start request from the terminal 10aa. More specifically, the management system 50 determines that the counterpart terminal 10bb is communicating, when the operation state "online (communicating)" is stored in association with the contact ID "01bb" of the counterpart terminal 10bb in the terminal management table of FIG. 6A.

At S126, the waiting data manager 58 of the management system 50 updates the waiting data management table of FIG. 6C, by adding an entry for the waiting terminal 10aa. The entry includes the contact ID "01bb" of the counterpart terminal 10bb, the contact ID "01aa" of the request terminal 10aa, the contact ID "01cc" of the referral terminal 10cc, and the received time when the management system 50 receives the communication start request from the terminal 10aa. The contact ID "01cc" of the referral terminal 10cc can be obtained from the referral data, which is received from the terminal 10cc at S122.

At S127, the waiting data manager 58 searches the waiting data management table of FIG. 6C, which is updated, using the contact ID "01bb" of the counterpart terminal 10bb as a search key to obtain the number of contact IDs of the waiting terminals 10. Further, based on the number of contact IDs of the waiting terminals 10, the waiting data manager 58 calculates a waiting time period. For example, a preset time period for consulting with one patient (for example, 7 minutes) may be multiplied by the number of waiting terminals.

At S128, the data transmitter/receiver 51 transmits the waiting data including the number of waiting terminals and the calculated waiting time period that are obtained at S 127, to the request terminal 10aa.

As the data transmitter/receiver 11 of the request terminal 10aa receives the waiting data, at S129, the display control 16 of the terminal 10aa displays various information based on the waiting data, such as the number of waiting terminals and the waiting time period.

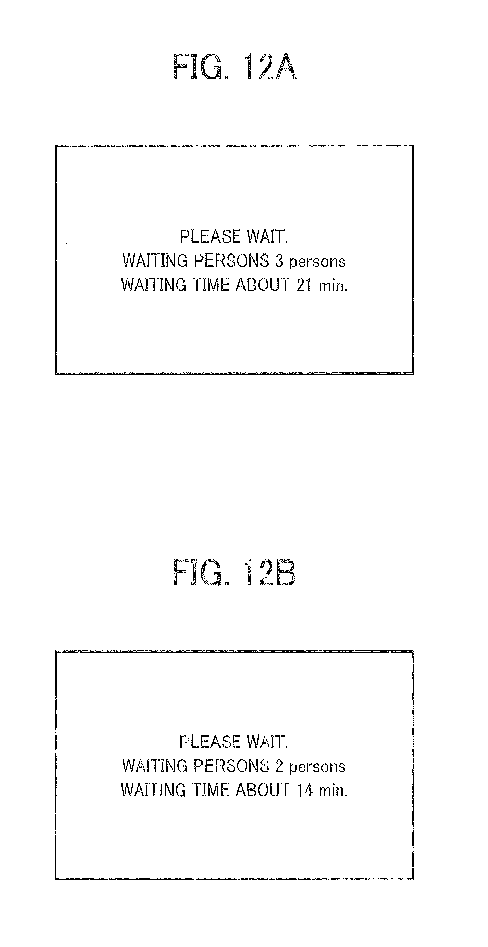

FIG. 12A is an example screen for display onto the display 120aa, at S129. With this information being displayed, the patient at the terminal 10aa is able to know the number of waiting terminals (the number of persons) and the waiting time, before starting communication with the terminal 10bb at the pharmacy. Since the waiting data, such as the waiting time, is notified to the waiting terminal 10aa, without starting a content data session "sed" between the terminal 10aa and the terminal 10bb, traffic on the communication network 2 can be suppressed.

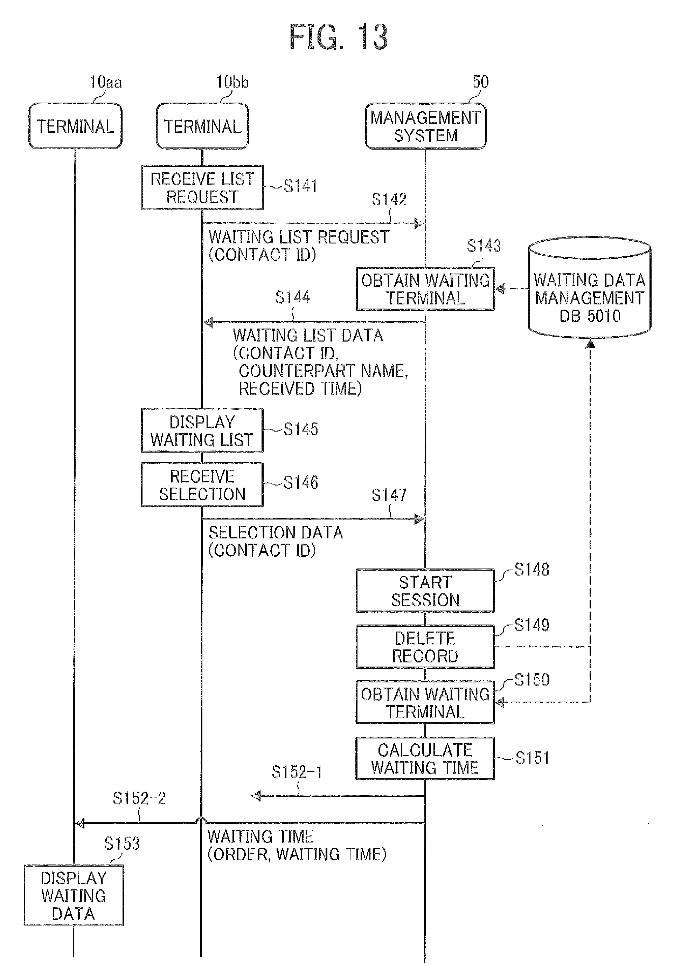

Referring to FIG. 13, operation of starting communication with the terminal 10aa, after ending a session with the other terminal 10, performed by the terminal 10bb, is explained according to an example embodiment of the present invention.

The pharmacist at the terminal 10bb presses the operation key 108 of the terminal 10bb, to request for a list of waiting terminals 10 that are waiting to start communication with the terminal 10bb. In response to such instruction, at S141, the operation input 12 of the terminal 10bb receives a request for waiting list.

At S142, the data transmitter/receiver 11 sends waiting list request information ("waiting list request") to the management system 50 through the communication network 2. The waiting list request information requests for a list of waiting terminals 10 that are waiting, and includes the contact ID "01bb" of the terminal 10bb.

As the data transmitter/receiver 51 of the management system 50 receives the waiting list request, at S143, the waiting data manager 58 searches the waiting data management table of FIG. 6C using the contact ID "01bb" of the terminal 10bb that requests for list as a search key, to obtain contact IDs of the waiting terminals, contact IDs of the referral terminals, and the received times. The data processor 59 further searches the terminal management table of FIG. 6A using the contact IDs of the waiting terminals and the referral terminals as a search key to obtain the terminal names of the waiting terminals and the terminal names of the referral terminals.

At S144, the data transmitter/receiver 51 sends the contact list data to the terminal 10bb that requests for list. The contact list data includes the contact IDs of the waiting terminals that are read at S143, the terminal names of the waiting terminals and the referral terminals, and the received times.

At S145, the contact list generator 20 of the request terminal 10bb generates a waiting list based on the waiting list data transmitted from the management system 50. More specifically, the contact list generator 20 lists the contact IDs of the waiting terminals, the terminal names of the waiting terminals, the terminal names of the referral terminals, and the received times, in an order such that a record with the earliest received time is listed first.

At S145, the display control 16 causes the display 120bb to display the waiting list.

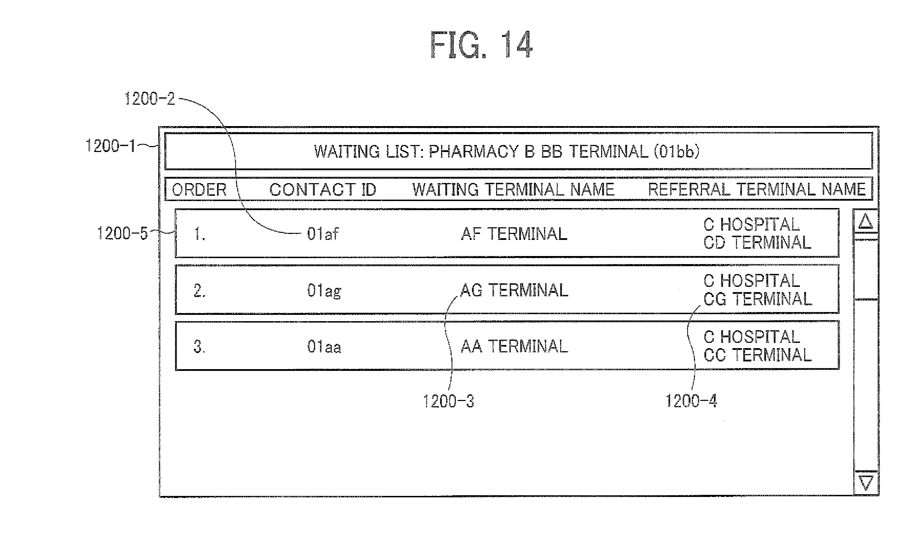

FIG. 14 illustrates an example waiting list screen. As illustrated in FIG. 14, within a waiting list frame 1200-1, the waiting list screen includes a plurality of keys 1200-5, each key displaying a contact ID 1200-2 of the waiting terminal, the terminal name 1200-3 of the waiting terminal, and the terminal name 1200-4 of the referral terminal, in association with a waiting order.

Referring back to FIG. 13, the pharmacist at the counterpart terminal 10bb selects, from among the waiting terminals displayed within the waiting list frame 1200-1, the waiting terminal 10af having the earliest received date, as a communication destination. At S146, the operation input 12 of the terminal 10bb receives selection of the terminal 10af as a communication destination of the operation input 12.

At S147, the data transmitter/receiver 11 of the terminal 10bb transmits selection data indicating selection of the terminal 10af as a communication destination, to the management system 50. The selection data includes the contact ID "01af" of the terminal 10af and the contact ID "01bb" of the terminal 10bb.

At S148, the management system 50 establishes a content data session "sed" between the terminal 10bb of the pharmacist and the terminal 10af of the patient. In establishing the session, the state manager 53 changes the operation state that is associated with the contact IDs"01af" and "01bb" of the terminals 10af and 10bb, in the terminal management table of FIG. 6A, respectively, from the "online (communication OK)" to "online (communicating). Further, in establishing the session, the data transmitter/receiver 51 requests the relay device 30 to start relaying of content data between the terminal 10af and the terminal 10bb. In this manner, the content data session "sed" is established between the terminal 10bb and the terminal 10af to start communication.

At S149, the waiting data manager 58 of the management system 50 deletes fields from the waiting data management table of FIG. 6C, with one field storing the contact ID "01bb" of the terminal 10bb, and the other field storing the contact ID "01af" of the terminal 10bb. Based on the updated waiting data management table of FIG. 6C, all waiting lists to be generated based on the waiting data management table of FIG. 6C will be updated for display at any terminal 10 that may be waiting. Further, when communication between the terminal 10bb and the terminal 10af ends, the pharmacist at the terminal 10bb is able to select a new terminal to start communication, based on the updated waiting list generated based on the updated waiting data management table of FIG. 6C.

At S150, the waiting data manager 58 searches the waiting data management table, which is updated, using the contact ID "01bb" of the counterpart terminal 10bb as a search key to obtain the contact IDs of the waiting terminals as well as the received times.

At S151, the waiting data manager 58 calculates, for each of the waiting terminals, a waiting time period based on the number of contact IDs of the waiting terminals and the received times. For example, the waiting terminals are arranged in an order determined by the received time. Then, the number of waiting terminals that are waiting before the subjected waiting terminal is multiplied by a preset time period (for example, 7 minutes).

At S152-1 and S152-2, the data transmitter/receiver 51 transmits waiting data including an order and the waiting time period calculated at S 151, to each of the waiting terminals 10ag and 10aa. As the data transmitter/receiver 11 receives the waiting data, at S153, the display control 16 causes the display 120 to display such as the order and the waiting time period.

FIG. 12B illustrates an example screen for display onto the display 120aa. As the screen of FIG. 12A is switched to the screen of FIG. 12B, the patient at the terminal 10aa is able to know that the number of waiting persons becomes two, and the waiting time period becomes less.

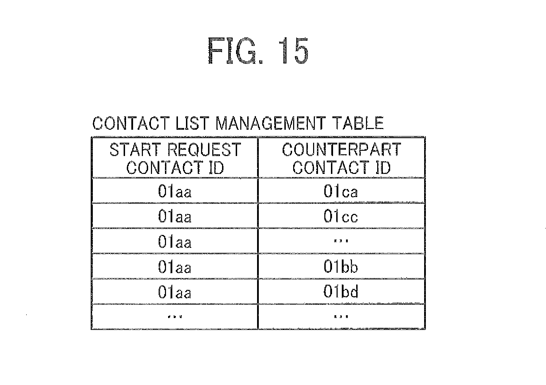

In the above-described example operation, the terminal 10cc of the doctor selects the terminal 10bb of the pharmacist, as a communication destination for the terminal 10aa of the patient. Alternatively, the terminal 10aa of the patient may select the terminal 10bb of the pharmacist to start communication with the terminal 10bb, for example, as described below referring to FIGs. 15 to 17.

In order to allow the terminal 10 of the patient to select the terminal 10 of the pharmacist, the contact list management table stores, for the terminal 10 of the patient, identification information for identifying one or more terminals 10 operated by the pharmacist. FIG. 15 illustrates a contact list management table according to this embodiment. The contact list management table of FIG. 15 stores, in association with the terminal ID "01aa" of the terminal 10aa operated by the patient, the contact ID of each one of the terminals 10ca, 10cb, etc. operated by the doctor, and the contact ID of each one of the terminals 10ba, 10bb, etc, operated by the pharmacist. Compared to the contact list management table of FIG. 6B, the patient at the terminal 10aa can select the terminal 10 operated by the pharmacist, in addition to the terminal 10 operated by the doctor.

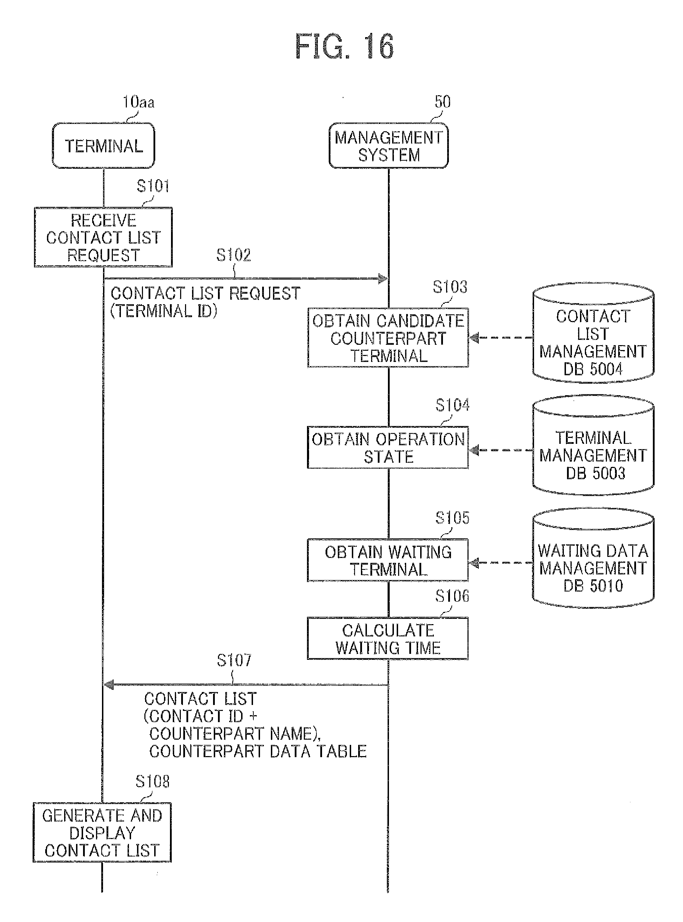

FIG. 16 is a data sequence diagram illustrating operation of preparing to start communication, performed by the terminal 10aa, according to this embodiment of the present invention. In this embodiment, the patient at the terminal 10aa requests for a contact list of pharmacists to the management system 50, and displays the contact list based on information received from the management system 50.

More specifically, at S101, the data transmitter/receiver 11 of the terminal 10aa sends contact list request information ("contact list request"), which requests for contact list, to the management system 50 through the communication network 2. The contact list request information includes the terminal ID "01aa" of the terminal 10aa ("request terminal 10aa").

In response to the contact list request, the management system 50 performs S103 to S107 in a substantially similar manner as described above referring to S103 to S107 of FIG. 8.

At S108, the contact list generator 20 of the request terminal 10aa generates a contact list based on the contact list information and the counterpart data table transmitted from the management system 50.

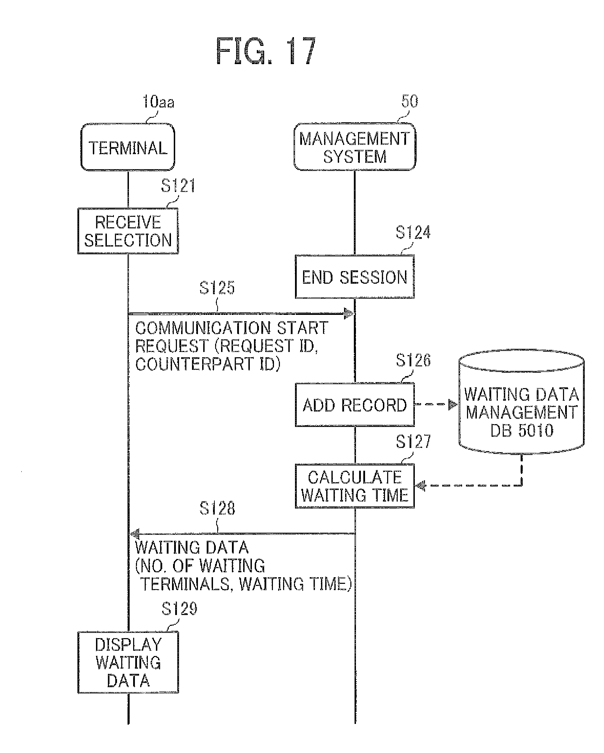

FIG. 17 is a data sequence diagram illustrating operation of starting communication with the counterpart terminal 10, which is elected from the contact list displayed through operation of FIG. 16, according to the embodiment of the present invention.

In this example, the patient at the terminal 10aa selects the terminal 10bb of the pharmacist, as a communication destination, to start communication with the terminal 10bb. In response to selection of the candidate counterpart terminal 10 of the pharmacy by the patient using the operation key 108, at S121, the operation input 12 of the terminal 10aa receives selection of the counterpart terminal 10bb.

At S124, the management system 50 sends the content data session "sed" between the terminal 10cc operated by the doctor and the terminal 10aa operated by the patient, for example, in response to a request for ending the session from the terminal 10cc or the terminal 10aa.

At S125, the data transmitter/receiver 11 of the terminal 10aa transmits communication start request information to the management system 50, which requests to start communication with the counterpart terminal 10bb. The communication start request information includes the contact ID "01aa" of the request terminal 10aa and the contact ID "01bb" of the counterpart terminal 10bb.

S126 to S129 are performed in a substantially similar manner as described above referring to S126 to S129 of FIG. 11.

As described above referring to FIGs. 15 to 17, the terminal 10aa of the patient is able to start communication with the counterpart terminal 10bb of the pharmacist, even without referral by the terminal 10bb of the doctor.

In any one of the above-described examples, without requiring a user (pharmacist) at the first communication terminal to enter information, a user (patient) at the second communication terminal that is waiting to start communication with the first communication terminal is able to obtain information regarding waiting, such as waiting time. More specilically, the management system 50 manages information regarding one or more waiting terminals, and sends waiting data based on the managed information to the waiting terminal.

The relay devices 30, the management system 50, the program providing system 90, and the maintenance system 100 in the above-described embodiment may be configured by a single computer or a plurality of computers to which divided units (functions) are arbitrarily allocated. In addition, in the case where the program providing system 90 is configured by a single computer, a program transmitted by the program providing system 90 may be separately transmitted in a plurality of modules, or may be transmitted in its entirety. Further, in the case where the program providing system 90 is configured by a plurality of computers, a program may be divided into a plurality of modules, and the modules may be individually transmitted from the respective computers.

In addition, a recording medium such as a CD-ROM storing the terminal program, the relay device program, or the communication management program in the above-described embodiment, the HD 204 storing these programs, and the program providing system 90 including the HD 204 are each used in the case where the terminal program, the relay device program, and the communication management program are provided as program products to users within a certain country or outside that country.

Further, although the IP address of the terminal 10 is managed in the terminal management table (see Fig. 6A) in the above-described embodiment, the embodiment is not limited to this case, and the fully qualified domain name (FQDN) of each terminal 10 may be managed instead as long as an FQDN serves as terminal identification information for identifying each terminal 10 on the communication network 2. In this case, an IP address corresponding to an FQDN is obtained by a Domain Name System (DNS) server of the related art.

In addition, although the case of a videoconference system has been described as an example of the communication system 1 in the above-described embodiment, the embodiment is not limited to this case, and the communication system 1 may be a car navigation system. In such case, the terminal 10 at one end corresponds to a car navigation system mounted on an automobile, and the terminal 10 at the other end corresponds to a management terminal or a management server at a management center or a car navigation system mounted on the other automobile.

The other examples of the communication system 1 include a teleconference system, a personal computer image sharing system, a telephone system including the Internet Protocol (IP) phone, Internet phone, or mobile phone. In the telephone system, the telephone operates as the communication terminal 10.

In addition, although image data and sound data are described as examples of content data in the above-described embodiment, the content data is not limited to these items of data, and the content data may be touch data. In this case, a feeling obtained by a user's contact at one terminal side is transmitted to the other terminal side. Further, the content data may be smell data. In this case, a smell at one terminal side is transmitted to the other terminal side. In addition, the content data may be at least one of image data, sound data, touch data, and smell data. More specifically, when the communication system 1 is used as a remote medical diagnosis system as described above, the content data may be sound data that reflects pulse or beam generated within a human body, image data of electrocardiogram, coordinate data that shows a trend in body temperature, or any other data that may be used to examine the patient.

In addition, although the case in which a videoconference is held by the communication system 1 has been described in the above-described embodiment, the embodiment is not limited to this case. The communication system 1 may be used in meetings, general conversation between family members or friends, or one-way presentation of information

Numerous additional modifications and variations are possible in light of the above teachings. It is therefore to be understood that within the scope of the appended claims, the disclosure of the present invention may be practiced otherwise than as specifically described herein. For example, elements and/or features of different illustrative embodiments may be combined with each other and/or substituted for each other within the scope of this disclosure and appended claims.

For example, in any one of the above-described embodiments, the contact list that lists one or more candidates of counterpart terminals, that is, one or more candidates of pharmacy, may be generated in various other ways. For example, the patient may want to select a specific pharmacy that the patient is familiar with. In such case, the management system 50 may previously store preference information indicating one or more pharmacies for the patient. Using the preference information, the contact list may be generated so as to display the preferred pharmacies on top, or only to display the preferred pharmacies.

In another example, the patient usually prefers to visit a pharmacy that is nearby. Based on this assumption, the management system 50 may generate the contact list so as to display the pharmacies that are closest to the patient location on top, or only to display the nearby pharmacies. For example, the management system 50 may calculate a distance between the pharmacy location and the patient location, and sorts the pharmacies by the calculated distance.

Further, any information relating to waiting may be output in various other ways, rather than displaying as a list. For example, such information may be output through a speaker as a message. Alternatively, the information being displayed is not limited to the above-described example such that only one of the number of waiting persons (or waiting terminals) and the waiting time may be displayed, or any additional information may be displayed.

Further, any of the above-described devices or units can be implemented as a hardware apparatus, such as a special-purpose circuit or device, or as a hardware/software combination, such as a processor executing a software program.

The present invention can be implemented in any convenient form, for example using dedicated hardware, or a mixture of dedicated hardware and software. The present invention may be implemented as computer software implemented by one or more networked processing apparatuses. The network can comprise any conventional terrestrial or wireless communications network, such as the Internet. The processing apparatuses can compromise any suitably programmed apparatuses such as a general purpose computer, personal digital assistant, mobile telephone (such as a WAP or 3G-compliant phone) and so on. Since the present invention can be implemented as software, each and every aspect of the present invention thus encompasses computer software implementable on a programmable device. The computer software can be provided to the programmable device using any conventional carrier medium. The carrier medium can compromise a transient carrier medium such as an electrical, optical, microwave, acoustic or radio frequency signal carrying the computer code. An example of such a transient medium is a TCP/IP signal carrying computer code over an TP network, such as the Internet. The carrier medium can also comprise a storage medium for storing processor readable code such as a floppy disk, hard disk, CD ROM, magnetic tape device or solid state memory device.

The hardware platform includes any desired kind of hardware resources including, for example, a central processing unit (CPU), a random access memory (RAM), and a hard disk drive (HDD). The CPU may be implemented by any desired kind of any desired number of processor. The RAM may be implemented by any desired kind of volatile or non-volatile memory. The HDD may be implemented by any desired kind of non-volatile memory capable of storing a large amount of data. The hardware resources may additionally include an input device, an output device, or a network device, depending on the type of the apparatus. Alternatively, the HDD may be provided outside of the apparatus as long as the HDD is accessible. In this example, the CPU, such as a cashe memory of the CPU, and the RAM may function as a physical memory or a primary memory of the apparatus, while the HDD may function as a secondary memory of the apparatus.

## Claims

1. A communication system (1), comprising:
a management system (50) communicable with a plurality of communication terminals (10) including a first communication terminal (10bb) and a second communication terminal (10aa) through a network (2), the management system (50) including:
a memory (5000) that stores waiting terminal identification information for identifying a waiting communication terminal, for each one of one or more waiting commmication terminals that are waiting to start communication with the first communication terminal (10bb); and
the second communication terminal (10aa) that sends a communication start request for starting communication between the first communication terminal (10bb) and the second communication terminal (10aa) to the management system (50),
wherein the management system (50) further includes:
a waiting data manager (58) that additionally stores identification information for identifying the second communication terminal (10aa) in the memory (5000), as the waiting terminal identification information of the waiting communication terminal; and
a transmitter (51) that transmits information related to waiting that is generated based on the waiting terminal identification information to the second communication terminal (10aa).

2. The communication system (1) of claim 1, wherein the waiting data manager (58) of the management system (50) further calculates a number of the one or more waiting communication terminals for the first communication terminal (10bb) based on the waiting terminal identification information stored in the memory (5000), the information relating to waiting including information indicating the calculated number of the one or more waiting communication terminals or a number of waiting persons obtained based on the calculated number of the one or more waiting communication terminals.

3. The communication system (1) of claim 1 or 2, wherein the waiting data manager (58) of the management system (50) further calculates a waiting time for the first communication terminal (10bb) based on the waiting terminal identification information stored in the memory (5000), the information relating to waiting including the calculated waiting time.

4. The communication system (1) of any one of claims 1 to 3, wherein the identification information of the waiting communication terminal includes at least one of:
identification information for identifying the waiting communication terminal; and
identification information for identifying a user at the waiting communication terminal.

5. The communication system (1) of any one of claims 1 to 4, further comprising:
a third communication terminal (10cc) that receives a selection of the first communication terminal (10bb) as a communication destination for the second communication terminal (10aa), and transmits identification information for identifying the first communication terminal (10bb) to the second communication terminal (10aa),
wherein the second communication terminal (10aa) transmits the communication start request to the first communication terminal (10bb) that is identified with the identification information received from the third communication terminal (10cc).

6. The communication system (1) of claim 5, wherein the third communication terminal (10cc) further transmits identification information for identifying the second communication terminal (10aa) to the management system (50).

7. The communication system (1) of claim 1, wherein the second communication terminal (10aa) includes:
an output interface (116, 117, 118) that outputs the information related to waiting through an output device.

8. The communication system (1) of claim 1, wherein, when the identification information of the waiting communication terminal is modified, the management system (50) further updates the information related to waiting based on the modification for transmission to the second communication terminal (10aa).

9. The communication system (1) of claim 1, wherein the communication system (1) is a remote medical diagnosis system.

10. A method of managing communication among a plurality of communication terminals (10), the method comprising:
storing in a memory, waiting terminal identification information for identifying a waiting communication terminal for each one of one or more waiting communication terminals that are waiting to start communication with a first communication terminal (10bb);
receiving (S125) a communication start request for starting communication between the first communication terminal (10bb) and a second communication terminal (10aa);
storing (S126) second identification information for identifying the second communication terminal (10aa) additionally in the memory, as the waiting terminal identification information of the waiting communication terminal; and
transmitting (S128) information relating to waiting that is generated based on the waiting terminal identification information to the second communication terminal (10aa).

11. The method of claim 10, further comprising:
calculating (S127) a number of the one or more waiting communication terminals for the first communication terminal (10bb) based on the waiting terminal identification information stored in the memory, the information relating to waiting including information indicating the calculated number of the one or more waiting communication terminals or a number of waiting persons obtained based on the calculated number of the one or more waiting communication terminals.

12. The method of claim 10 or 11, further comprising:
calculating (S127) a waiting time for the first communication terminal based on the waiting terminal identification information stored in the memory, the information relating to waiting including the calculated waiting time.

13. The method of any one of claims 10 to 12, wherein the identification information of the waiting communication terminal includes at least one of:
identification information for identifying the waiting communication terminal; and
identification information for identifying a user at the waiting communication terminal.

14. A carrier means carrying computer readable code for controlling a computer to carry out the method of any one of claims 10 to 13.

15. A management system (50) configured to perform the method of any one of claims 10 to 13.
